(19) Europäisches Patentamt
European Patent Office
Office européen des brevets

(11) Publication number: **0 298 516 A2**

(12) # EUROPEAN PATENT APPLICATION

(21) Application number: 88110977.1

(22) Date of filing: 08.07.88

(51) Int. Cl.4: C07H 15/203 , C12Q 1/34 , //C09B69/10

(30) Priority: 08.07.87 JP 170469/87

(43) Date of publication of application: 11.01.89 Bulletin 89/02

(84) Designated Contracting States: DE GB

(71) Applicant: FUJI PHOTO FILM CO., LTD.
210 Nakanuma Minami Ashigara-shi
Kanagawa 250-01(JP)

(72) Inventor: Osada, Chiaki

Deceased(JP)

(74) Representative: Patentanwälte Grünecker, Kinkeldey, Stockmair & Partner
Maximilianstrasse 58
D-8000 München 22(DE)

(54) **Azo dyestuff glycosides and a method for their manufacture.**

(57) An azo glycoside composition containing a compound represented by formula (I):

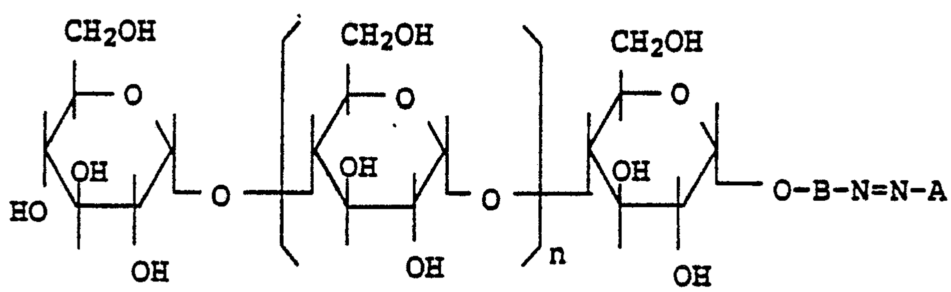

(I)

where A represents a substituted or unsubstituted phenyl group or a substituted or unsubstituted naphthyl group; B represents a substituted or unsubstituted phenylene group; and n represents zero or an integer of 1 to 8. The azo glycoside composition is useful for measurement of α-amylase without interference by pigments in the blood.

EP 0 298 516 A2

# AZO DYESTUFF GLYCOSIDES AND A METHOD FOR THEIR MANUFACTURE

## FIELD OF THE INVENTION

The present invention relates to azo dyestuff glycosides which are useful in the measurement of the activity of polysaccharide hydrolases, such as amylase, and also to a method of manufacturing such azo dyestuff glycosides.

## BACKGROUND OF THE INVENTION

Oligosaccharide derivatives in which an aromatic ring which has characteristic absorption in the ultraviolet or visible light wavelength regions, e.g. phenol or p-nitrophenol, etc. is bonded at the α-position in the glucose unit are known, and are described in Nature 182, pages. 525-526 (1958); Journal of Biochemistry (Tokyo) 62, No. 4, pages 439-446 (1967); Carbohydrate Research 2, No. 5, pages 418-420 (1966); JP-A-53-12381 (The term "JP-A" as used herein means an "unexamined published Japanese patent application") and JP-A-54-51892, etc. These substrates are useful for the measurement of the activity of polysaccharide hydrolases such as maltase, glucoamylase, and amylase, etc., in biological liquids such as blood. However, when α-amylase activity is measured using such a substrate, the measurement of light absorption has to be carried out at a fairly short wavelength, namely in the vicinity of 410 nm, and if a substance such as bilirubin which absorbs light at short wavelengths is also present in the sample liquid such as blood serum, there will be ready susceptibility to interference by this substance. In order to eliminate such interference, it is necessary to carry out a "blank test", and this makes the analytical operation complex.

## SUMMARY OF THE INVENTION

An object of the present invention is to provide compounds useful as self-developing substrates which, in the measurement of α-amylase activity, are not readily susceptible to interference by colored substances in the blood such as bilirubin and hemoglobin.

Another object of the invention is to make possible measurment of α-amylase activity by a simple analytical operation with high detection sensitivity.

A further object of the invention is to provide a method of manufacturing such compounds.

A further more object of the invention is to provide an analytical element for the measurement of the activity of polysaccharide hydrolases.

It has now been found that these and other objects of the present invention can be attained by an azo glycoside composition containing a compound represented by formula (I):

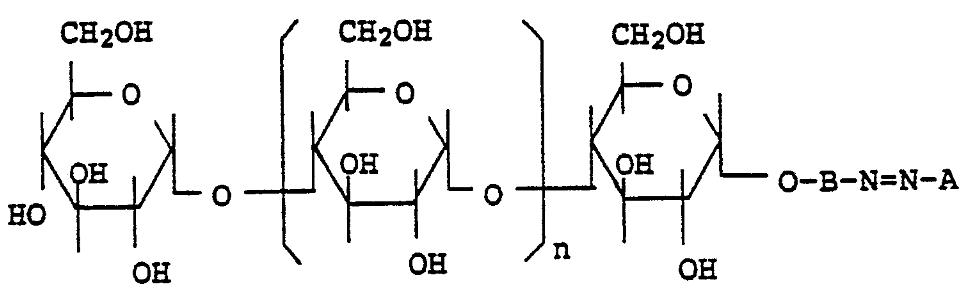

(I)

where, A represents a substituted or unsubstituted phenyl group or a substituted or unsubstituted naphthyl group; B represents a substituted or unsubstituted phenylene group; and n represents zero or an integer of 1 to 8. The invention also relates to a method for producing these compounds, and further relates to an analytical element for measuring activity of polysaccharide hydrolases using these compounds.

## DETAILED DESCRIPTION OF THE INVENTION

The phenyl or naphthyl groups represented by A in general formula (I) may have one or more substituent groups, including for example, halogen atoms (e.g., chlorine atoms), nitro groups, alkane sulfonyl groups (where the number of carbon atoms is preferably no more than 4), sulfonamido groups or sulfamoyl groups (where the number of carbon atoms is preferably from 1 to 7), alkyl groups (where the number of carbon atoms is preferably no more than 4), alkoxy groups or alkylthio groups (where the number of carbon atoms is preferably no more than 4, and each group may be substituted with a further alkoxy group), phenoxy groups (which may have halogen or other substituents), or phenylthio groups.

The phenylene group represented by B in general formula (I) is preferably a 1,4-phenylene group, and these may have one or more substituents. The substituent groups may be, for example, alkyl groups (where the number of carbon atoms is preferably no more than 4), alkoxy groups or alkylthio groups (where the number of carbon atoms is preferably no more than 4, and these groups may be substituted with a further alkoxy groups), sulfonamide groups or sulfamoyl groups (where the number of carbon atoms is preferably no more than 7), carbonamido groups or carbamoyl groups (where the number of carbon atoms is preferably no more than 8) or hydroxyl groups.

In formula (I), n is preferably 3, 4 or 5.

In the method according to the invention an olgiosaccharide represented by general formula (Ia):

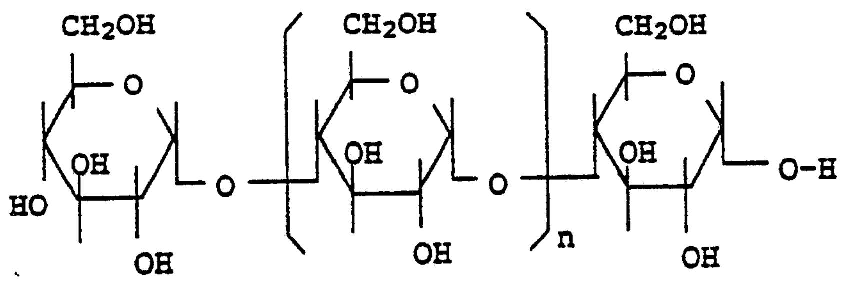

(Ia)

is reacted with an organic anhydride represented by formula (Ib):

$(RCO)_2O$ (Ib)

(where R represents a lower alkyl group), to produce the compound represented by formula (Ic):

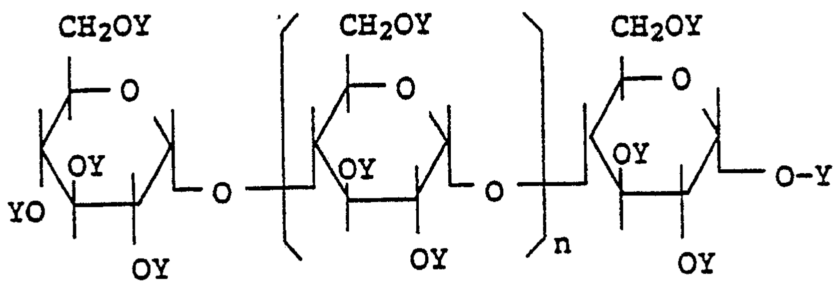

(Ic)

(where Y represents COR); the compound represented by formula (Ic) obtained is halogenated to produce a compound represented by formula (Id):

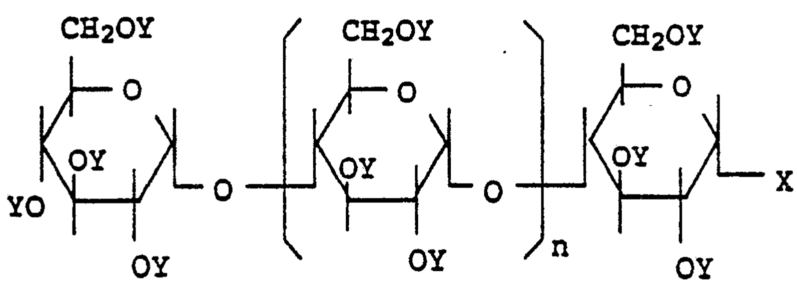

(Id)

(where X represents a halogen atom), and compound (Ie) is then an azo dyestuff represented by formula (Ie)

HO-B-N = N-A (Ie)

to produce a compound represented by formula (If) is obtained:

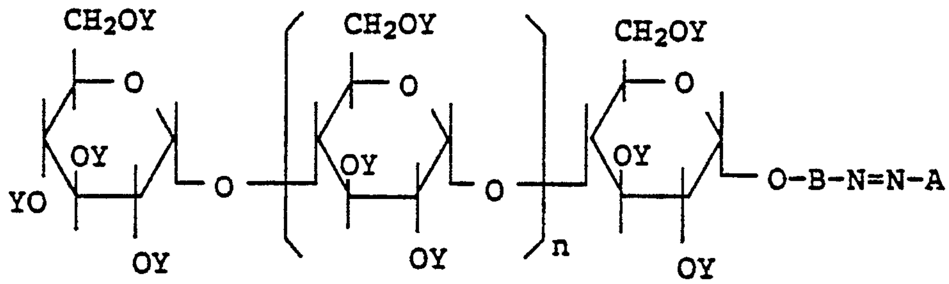

(If)

which is then deacrylated to produce the compound represented by formula (I).

Among the compounds represented by general formula (I), compounds with outstanding stability are those where -B-N = N-A is represented by formula (II):

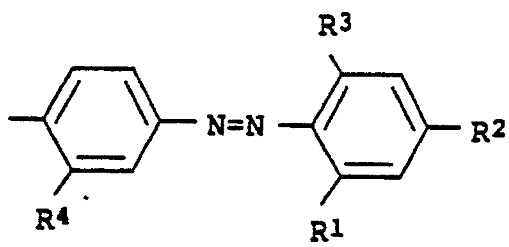

(II)

where $R^1$ and $R^2$ which may be the same or different, each represents a hydrogen atom, a chlorine atom, a bromine atom, a nitro group, a methane sulfonyl group, or an ethane sulfonyl group, $R^3$ represents a hydrogen atom, a chlorine atom or a bromine atom, and $R^4$ represents a hydrogen atom, a chlorine atom, a bromine atom or lower alkyl group (methyl, ethyl or other group with at most 4 carbons). In the case of compounds where at least one of the two groups $R^1$ and $R^2$ is a nitro group, methane sulfonyl group or ethane sulfonyl group, the dyestuff released as a result of hydrolysis has an absorption maximum at a comparatively long wavelenght and further, the molecular extinction coefficient is high.

Other preferable compounds are those where -B-N = N-A is represented by general formula (III)

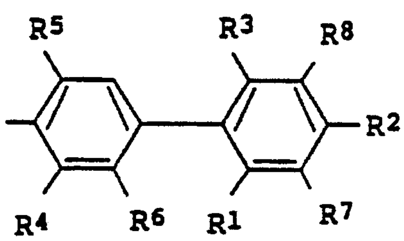

(III)

wherein $R^1$, $R^2$, $R^3$ and $R^4$ each presents the same meaning as defined for that in general formula (I), $R^5$ represents the same meaning as defined for $R^4$. $R^7$ and $R^8$ each represents the same meaning as defined for $R^1$. $R^6$ represents a hydrogen atom, a halogen atom or $-NHCOR^{6'}$ ($R^{6'}$ represents a lower alkyl group having 1 to 4 carbon atoms or a phenyl group.)

The compounds represented by formula (I) differ from p-nitrophenylpentaose or p-nitrophenylheptaose which are substrates conventionally widely used in the measurement of α-amylase activity, and the absorption maximum of the dyestuff released is at a high wavelength, so that it is not readily susceptible to interference by the bilirubin or hemoglobin, etc. in the blood.

Specific examples of the compounds represented by general formula (I) are shown below, where Q represents

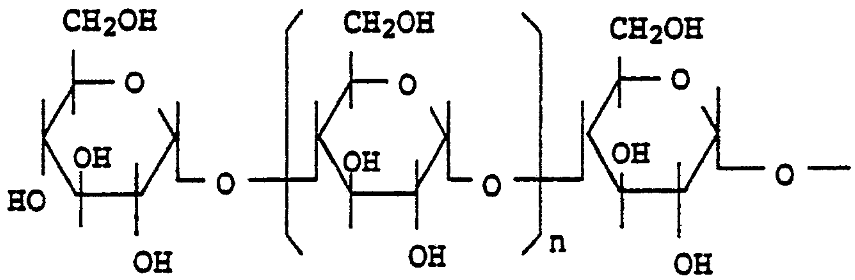

and n is 3 or 5.

(1)

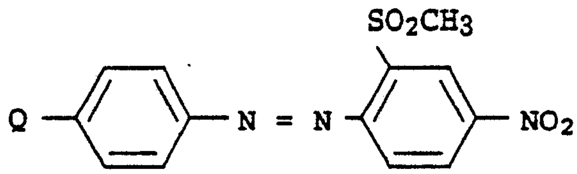

(2)

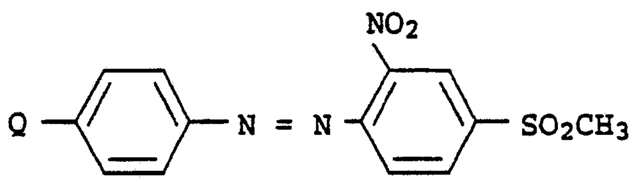

(3)

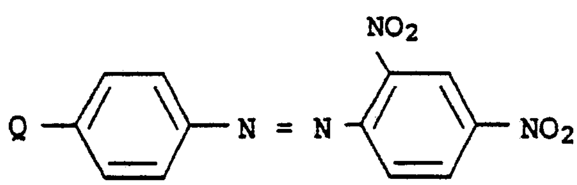

(4)

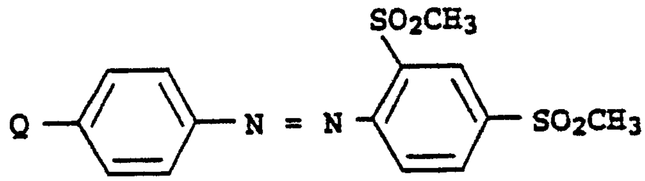

(5)

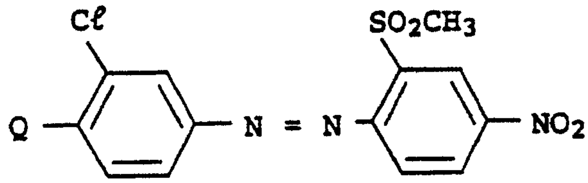

(6)

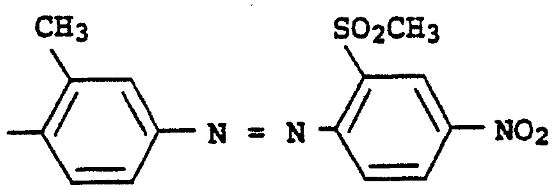

(7)

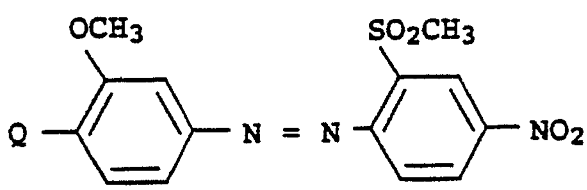

(8)

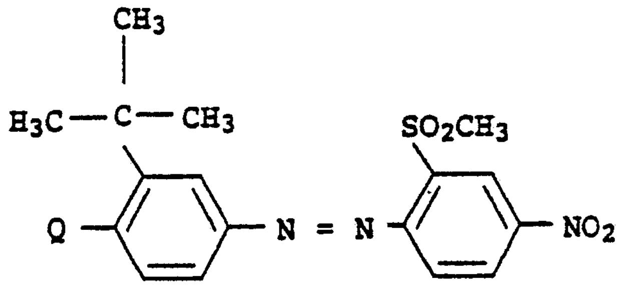

(9)

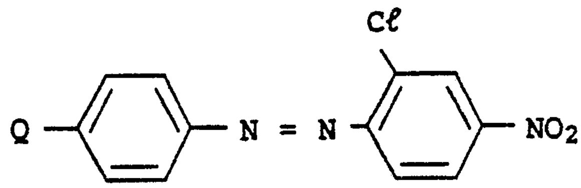

(10)

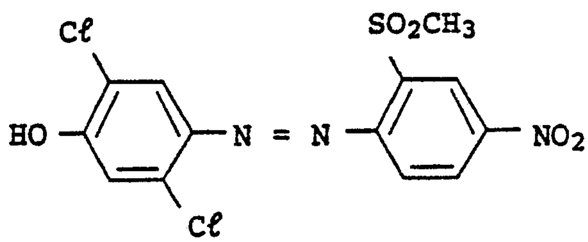

(11)

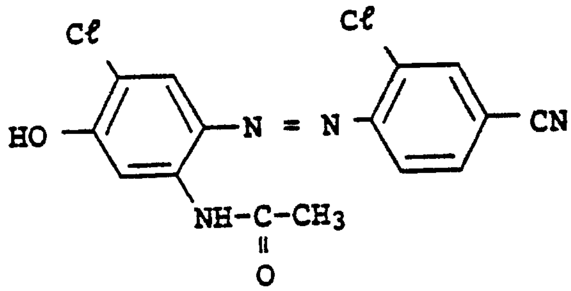

(12)

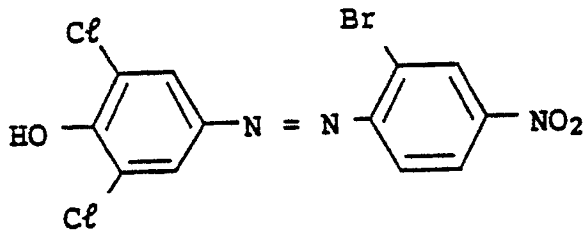

(13)

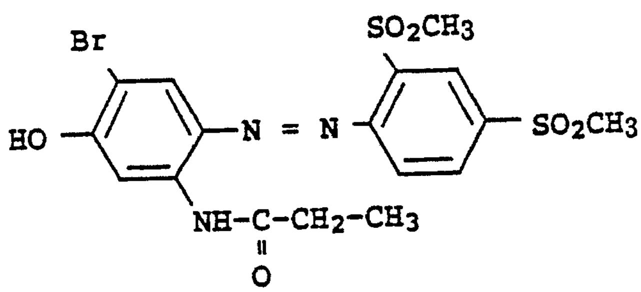

(14)

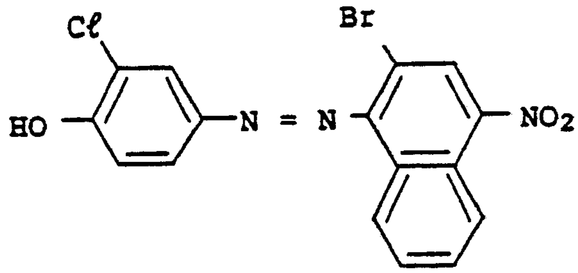

(15)

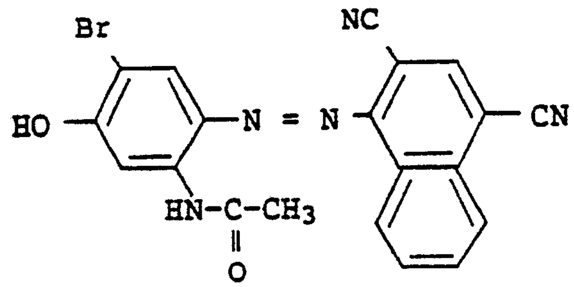

Each of the individual reactions in the method of manufacturing the compounds of the present invention will now be explained in greater detail.

Acylation of the hydroxyl groups:

The acylation of the oligosaccharide can be carried out by a known method, for example by treatment in an organic anhydride as the reactant, preferably in the presence of an anhydrous alkali metal salt of an organic acid or other such catalyst while heating.

The organic anhydride represented by $(RCO)_2O$ can be, for example, acetic anhydride, propionic anhydride or butyric anhydride, etc. Examples of the catalyst used are anhydrous alkali metal salts of organic acids (such as acetic acid and propionic acid), such as their sodium or potassium salts, and pyridine or picoline, etc.

In order to facilitate the control of the reaction or the purification of the product following reaction, it is possible to add a non-aqueous solvent such as chloroform or dichloromethane to the reaction solution.

The amount of organic anhydride used in the aforesaid reaction is about 5 to 50 times the weight of oligosaccharide, preferably about 7 to 15 times. Further, in the case where an anhydrous alkali metal salt of an organic acid is used as the catalyst, the amount of catalyst is about 0.5 to 3 times the weight of the oligosaccharide, preferably about 0.5 to 1.5 times.

The reaction temperature will normally be about 90 to 140 °C, preferably about 100 to 110 °C. The reaction time will be influenced by the reaction temperature, but under the preferred reaction temperature conditions it will be about 2 to 4 hours. The reaction mixture is cooled to 0 to 5 °C by a usual method, and the solid material deposited is separated off, washed with water and then dried. The solid product obtained can be recrystallized using solvents such as ethanol, methanol or other alcohol, methyl ethyl ketone, acetone or other ketone, dimethyl ether, diethyl ether or other ether, etc. either alone or in combination, but the solid product after drying sufficiently can also be used for the subsequent reaction without recrystallization.

Halogenation of the terminal:

The halogenation is carried out with anhydrous hydrogen halide, aluminium chloride, phosphorus pentachloride, or titanium tetrachloride, stannic chloride, but from the point of view of product yield, the suppression of side reactions (which relates to the product yield) and the ease of purification of the desired product, a treatment method using anhydrous titanium tetrahalide in a non-aqueous solvent of low polarity such as chloroform or dichloromethane, etc. is particularly preferred.

Titanium tetrachloride, titanium tetrabromide and titanium tetraiodide, etc. can be used as the anhydrous titanium tetrahalide, and the amount of this anhydrous titanium tetrahalide will normally be about 1 to 20 times the molar proportion of the acylated oligosaccharide, preferably about 3 to 8 times.

The halogenation reaction can be carried out at normal pressure between room temperrture and the boiling point of the solvent used, but is particularly preferred that the reaction be carried out while refluxing at the boiling point of the solvent. The reaction time is influenced by the reaction temperature but when the reaction is performed near the solvent boiling point, it will normally be from about 30 minutes to 1 hour.

The reaction mixture is cooled by a usual method, an organic solvent such as chloroform, dichloromethane, or ethyl acetate is added, and then the organic solvent phase is separated. After washing a number of times with water or saturated aqueous sodium bicarbonate solution, then the solvent is eliminated to complete dryness.

The solid product obtained can be purified by separation using silica gel chromatography or other such conventional method, followed by recrystallizing from solvents such as ethanol, methanol or other such alcohol, methyl ethyl ketone, acetone or other such ketone, dimethyl ether, diethyl ether or other such ether, etc., used either alone or in combination, but it is also possible to use the solid product without purification in the next reaction after sufficient drying.

Substitution Reaction:

The anomeric halogen group in the aforesaid halogenated compound (Id) is substituted by an azo dyestuff of aforesaid general formula (Ie), thus (If) is obtained.

The amount of the azo dyestuff used in this reaction is about 1 to 20 times the molar amount of compound (Id), preferably about 1.2 to 3.0 times.

The azo dyestuff needs to dissociate forming a salt in the reaction solvent for the reaction to proceed, and so an inorganic salt of the azo dyestuff, e.g., the sodium salt, potassium salt or barium salt, or an organic salt such as the triethylamine salt, tributylamine salt, pyridine salt or picoline salt, etc. is used. It is possible to employ two or more such salts together. Moreover, the salt of the azo dyestuff need not be prepared beforehand, and an inorganic base or organic base may be added to the reaction solvent, or alternatively, an organic base may be used directly as the reaction solvent. The amount of base added will preferably be an amount sufficient to maintain the liquid under neutral or alkaline conditions up to the completion of the reaction.

It is usually preferred that this reaction be carried out in the presence of a solvent. The solvent employed is not particularly restricted as long as it does not participate in the reaction, but a solvent in which the halogenated compound (Id) and the azodyestuff (Ie), or the azo dyestuff salt, have high solubility, and which will enhance the reactivity is preferred. For example, the following solvents may be employed: amides such as dimethyl formamide, dimethyl acetamide, etc., nitriles such as acetonitrile, benzonitrile, etc., dimethyl sulfoxide, organic bases such as trialkylamines, pyridine, lutidine, etc., and aromatic hydrocarbons such as benzene and toluene. Further, mixtures of two or more such solvents can be used.

This reaction will, generally speaking, proceed over the range about -5 to 100 °C but normally a reaction temperature of about 10 to 50 °C is preferred.

The reaction time will differ according to the type of base used as a reaction auxiliary and the reaction temperature, but normally, it will be about 5-20 hours. At the completion of the reaction, the reaction mixture is poured into ice water and the solid material deposited is filtered off, or alternatively, the desired product is extracted with an appropriate organic solvent, then dried, and the solvent eliminated to give a solid product. Compound (If) is obtained as a solid material.

This product can be purified in any conventional way, for example, by column chromatography using alumina or silica gel, etc., a crystallization method employing an organic solvent,etc., or optionally, by their combination.

De-acylation reaction:

The removal of the acyl groups from compound (If) can be carried out by known methods, for example, in the presence of a catalyst such as an alkali metal alkoxide in dehydrated methanol or a methanol solution of anyhydrous ammonia. As the alkali metal alkoxide, there can be used, for example, sodium methoxide, potassium methoxide, sodium ethoxide, potassium ethoxide, potassium t-butoxide, or the like.

It is preferred that the reaction be carried out following the addition of a non-aqueous solvent of low polarity such as chloroform or dichloromethane to the dehydrated methanol, in order to facilitate the purification of the desired product after the end of the reaction. The non-aqueous solvent of low polarity added is useful to precipitate the azo dyestuff glycoside produced from the reaction system, without inhibiting the de-acylation reaction. The amount of non-aqueous solvnet added will vary according to the solvent, about 0.5 to 2 times of dehydrated methanol by volume being preferred.

The de-acylation reaction will be complete within about 6-24 hours at a temperature of about 0-30 °C. In a reaction system in which dry methanol is the sole solvent, the methanol will be distilled away under reduced pressure after the completion of the reaction. Then, the solid material obtained is treated using an acidic ion-exchange resin or an inorganic acid to neutralize the basic material present, after which the compound is purified by thin layer chromatography or column chromatography, etc. In the case of a reaction system to which a solvent of low polarity has been added, the desired product will be precipitated from the reaction liquid. Hence, it is filtered off, and may then be subjected to a separation/purification process.

The analytical element and the analytical method using the composition of this invention can overcome various problems encountered in conventional whole blood analysis. That is, according to this invention, the analysis can be carried out easily, quickly, and accurately and also an undiluted whole blood sample can be analyzed. Further, in this invention, it is unnecessary to separate previously the serum or plasma from the other cell components of the whole blood.

Since the dye formed by the interaction with the assay component is detected by a spectrophotometer at a wavelength of 500 nm or longer, the disturbances caused by homoglobin in blood can be avoided.

The analytical method using the composition of this invention is very quick. That is, the analysis can be usually finished within several minutes or, in some cases, within 2 minutes.

This invention is useful for the measurement of enzyme activity of hydrolase (e.g., amylase, etc.).

The invention can be also useful for an immunological assay involving a specific antibody or antigen by means of an enzyme-labelled antigen or enzyme-lebelled antibody.

A dry system multilayer analytical element using the composition of this invention comprises at least two water-permeable layers having each different functions, that is (1) a liquid spreading layer which can receive or absorb a whole blood sample (of e.g., 1 to 20 $\mu\ell$) and does not need the wiping off of excessive blood and (2) a color-forming layer (reagent layer) for forming a dye by way of a reaction with a component to be determined. These two layers respectively may be composed of two or more layers. Also, the analysis element may further have a light-reflection layer or a corpuscle filter layer as described in U.S. Patent 4,042,335.

The liquid spreading layer is composed of a fibrous or non-fibrous material having a proper porosity and mean pore size capable of absorbing whole blood or a mixture of these materials as is disclosed in U.S. Patent Nos. 3,992,158 and 4,292,272. It is preferred that the liquid spreading layer provides whole blood of uniform cocentration per unit area to the surface of another water-permeable layer, including reagent layer, adjacent to the liquid spreading layer in liquid contact relation.

The useful liquid spreading layer is composed of a cloth described in U.S. Patent No. 4,292,272 and fibrous material such as a knitting described in JP-A-60-222769. Also, the liquid spreading layer may be composed of a non-fibrous isotropic porous material (e.g., a blush polymer) described in U.S. Patent 3,992,158.

The liquid spreading layer of this invention may also function to filter off substantially all of or at least a part of the corpuscles in blood. The aforesaid cloth or knitting is useful for this purpose.

The liquid spreading layer in this invention may contain a hydrophilic polymer or a surface active agent as described in JP-A-60-222770, Japanese Patent Application Nos. 61-122875, 61-122876, and 61-143754, for controlling the liquid spreading area, the spreading speed, etc.

The reagent layer of the dry analytical element according to the present invention may contain, if necessary, a hydrophilic polymer, a buffer agent, light-shielding fine particles (either reflective or absorbing), and the like.

The hydrophilic polymer which can be used in the reagent layer includes starch, cellulose, derivatives thereof (e.g., hydroxyethylated derivatives, hydroxypropylated derivatives, etc.), agarose, gelatin acrylamide

polymers, copolymers of acrylamide and various vinyl monomers, polyvinyl alcohol, copolymers of vinylpyrrolidone and various vinyl monomers, acrylate polymers, copolymers of acrylates and various vinyl monomers, and the like. Of these hydrophilic polymers, polyvinyl alcohol, vinylpyrrolidone polymers, acrylamide polymers and cellulose derivatiaves are preferred.

The reagent layer and/or the liquid spreading layer of the analytical element contains the composition of this invention. The composition may contain at least one active component which causes an interaction with an assay component in a liquid sample to release an enzyme which hydrolyse the composition of this invention, when a liquid sample containing the assay component is brought into contact with the reagent layer or the liquid spreading layer.

The feature of the analytical element or the analytical method using the composition of this invention is that a dye capable of being detected by a spectrophotometer at a wavelength of 500 nm. or longer is formed directly or indirectly by the aforesaid interaction. Dyes released from the composition of this invention through hydrolysis have sufficiently high absorbance that the significant optical density thereof can be observed at a wavelength of 500 nm or longer.

The dye released from the composition of this invention may be a diffusible dye capable of being transferred from the reagent layer or the spreading layer into a detection layer containing no reagent, though it may be non-diffusible.

The detection layer is a layer which is substantially permeable to a substance to be finally detected and contains substantially no reagent. The layer comprises a hydrophilic polymer as a main component and may comprise, if desired, a surface active agent (cationic, amphoteric or nonionic), a hardener, a buffer agent, etc.

The hydrophilic polymer which can be used for the detecting layer is a natural or synthetic hydrophilic polymer usually having a degree of swelling of from about 1.5 to about 20, and preferably, from about 2.5 to about 15, when it absorbs water.

All of the reagent composition (interacting composition) may be contained in the reagent layer. In an other embodiment, one component or more of the reagent composition may be contained in the reagent layer and the remainder is contained in the liquid spreading layer. For instance, a composition releasing hydrolase such as amylase by the reaction of an assay component to be determined with a reagent in the composition may be contained in the liquid spreading layer and the composition of this invention producing a dye may be contained in the reagent layer. Interferring substance-eliminating layer, as is disclosed in U.S. Patent 4,066,403 may be interposed between the reagent layer and the liquid spreading layer.

The reagent layer may be composed of a fibrous porous material such as a filter paper, a non-woven fabric etc., or a non-fibrous porous material. The reagent layer preferably comprises cellulose esters as is disclosed in JP- B-58-21677, U.S. Patent 1,421,341, etc., and more preferably comprises a blushed polymer consisting of such as cellulose acetate, cellulose acetate/cellulose butyrate, cellulose nitrate, etc. In addition, the reagent layer may be composed of a microporous film of a polyamide, such as 8-nylon, 6,6-nylon, etc., a polyethylene, a polypropylene, polysulfone as is disclosed in JP-B-58-21677, etc. Further, the reagent layer may comprise a polymer particle-structure as is disclosed in JP-A-57-101760 and 57-101761 and a porous material having continuous voids wherein polymer particles, glass particles, diatomaceous earth, etc. are bonded using hydrophilic or non-water absorbing polymer.

The practical layer construction of the dry-analytical element of this invention using a light-transmitting support are as follows.

(1) An analytical element composed of a support having formed thereon, a reagent layer, and a superposed liquid spreading layer which may have further function as a corpuscle filter layer.

(2) An analytical element composed of a support having formed thereon, in succession, a detection layer, a reagent layer, and a liquid spreading layer which may have further function as a corpuscle filter layer.

(3) An analytical element composed of a support having formed thereon, in succession, a reagent layer, a light reflection layer, and a liquid spreading layer, one or more of said layers may have a further function as a corpuscle filter layer.

(4) An analytical element composed of a support having formed thereon, in succession, a detection layer, a reagent layer, a light reflection layer, and a liquid spreading layer.

(5) An analytical element composed of a support having formed thereon, in succession, a detection layer, a light reflection layer, a reagent layer, and a liquid spreading layer (said light reflection layer or liquid spreading layer may have a further function as a corpuscle filter layer).

(6) An analytical element composed of a support having formed thereon, in succession, a detection layer, and a liquid spreading layer containing the composition of this inveniton (said liquid spreading layer may have a further function as a corpuscle filter layer).

(7) An analytical element composed of a support having formed thereon, in succession, a detection layer, a light reflection layer, and a liquid spreading layer containing the composition of this invention, one of said layers may have a further function as a corpuscle filter layer.

In elements (1) to (7) described above, a water absorbing layer may be formed between the support and the reagent layer or the detection layer.

The water-absorbing layer is a layer which is permeable to water but substantially impermeable to the substance to be finally detected. This layer is provided, preferably between a support and a detecting layer or a reagent layer, in cases where the dye to be detected is a sparingly diffusible substance. Such a water-absorbing layer preferably comprises a film-forming hydrophilic polymer which is swollen with absorbed water. When in analytical elements (1), (2) and (5) described above, the liquid spreading layer or the light reflection layer does not have a function as a corpuscle filter layer, a corpuscle filter layer may be formed between the reagent layer and the liquid spreading layer. Also, when in analytical elements (3) and (4), the liquid spreading layer or the light reflection layer do not have a function as a corpuscle filter layer, a corpuscle filter layer may be formed between the reagent layer and the detection layer or between the liquid spreading layer or the reagent layer and the light reflection layer. Furthermore, when in analysis element (5) described above, the liquid spreading layer or the light reflection layer do not have a funcion as a corpuscle filter layer, a corpuscle filter layer may be formed between the reagent layer and the light reflection layer.

Likewise, when in analysis elements (6) and (7) described above, the liquid spreading layer or the light reflection layer do not have a funcion as a corpuscle filter layer, a corpuscle filter layer may be formed between the detection layer or the light reflection layer and the liquid spreading layer.

The analytical element of this invention may have a layer of substantially filtering off all corpuscles apart from the liquid spreading layer. Examples of such a layer include the porous layers described in JP-A-58-70163, 61-4959, 62-116258, 62-138756, 62-138757 and 62-138758.

The analytical element of this invention may have a light reflection layer as described above. Also, the liquid spreading layer or corpuscle filter layer may be provided with a fuction as a light reflection layer. The light reflection layer is a layer which functions to shield the color of a sample liquid being analyzed when spotted onto the liquid spreading layer and, in particular, the red color of hemoglobin and the yellow color of bilirubin, when measuring the detectable change formed in the reagent layer or in the detection layer by measuring light reflected from the layer, and functions as a light-reflecting or background layer at the same time.

It is preferred that the light reflection layer is a water-permeable layer composed of a hydrophilic polymer as binder having dispersed therein light reflective fine particles such as particles of titanium oxide, barium sulfate, etc. As the binder, gelatin, an acid derivative of gelatin, polyacrylamide, etc., are preferred. In the case of using a hardenable polymer such as gelatin, a hardening agent may be added thereto. Light reflecting particles of titanium oxide, etc., may also be incorporated into the liquid spreading layer, the reagent layer, the corpuscle filter layer, the detection layer, etc., for giving light-relfecting function to such a layer.

One or more layers of the analytical element of this invention may further contain at least one of various additives such as a surface active agent, a solvent for color-forming agent, a buffer, a binder, a hardening agent, etc., in an amount known in the field of art. Typical examples of these additives are described in U.S. Patents 4,258,011, 3,992,158, 4,042,335, 4,144,306, 4,132,528, 4,050,898, 4,275,152, and JP-A-62-299.

After spotting a whole blood sample to the analytical element, the analytical element can be subjected to incubation (warming) for obtaining the test result quickly and accurately.

When an assay component is present in the analytical elemnt, the assay component causes an interaction with the interacting composition at a rate depending on the concentration of the assay component in the sample. The formation rate of the dye or the amount of dye formed corresponding to the concentration of the assay component is measured by passing the analytical element through a proper apparatus for detecting the dye. The dye can be detected quantitatively using a spectrophotometer as described, for example, in U.S. Patent 4,584,275.

The invention is explained more practically by the following examples.

In the following examples, methods of manufacturing some typical compounds according to the invention are described in greater detail, but the present invention is not to be construed as being limited thereto. It will also be easy to synthesize compounds other than those described below using the procedures given below.

Unless otherwise indicated, all parts, percents and ratios are by weight.

## EXAMPLE 1

(Method of manufacturing 2-chloro-2′-methane sulfonyl-4′-nitro-azobenzene maltopentaoside)

(A) Method for manufacturing heptadecaacetylmaltopentaose

A mixture of 20 g of maltopentaose, 260 ml of acetic anhydride and 20 g of anhydrous sodium acetate was stirred for 5 hours at 100-110° C, then poured into 600 ml of ice water and stirred for 10 hours. After cooling to 5° C crystallization occurred, after which the product was filtered off, washed with water and dried. The crystals obtained were recrystallized two times from ethanol and 35 g of the desired product was obtained.
Melting point = 123-127° C.

B) Method of manufacturing hexadecaacetylmaltopentaosyl chloride

10 g of the heptadecaacetylmaltopentaose obtained in (A), 50 mℓ of dry chloroform and 6 g of titanium tetrachloride were reacted under reflux for 1 hour while stirring. Following the reaction, 300 ml of chloroform was added, and after washing three times with 100 ml of water, the chloroform layer was separated and then 30 g of anhydrous sodium sulfate was added to eliminate any moisture. Next, the anhydrous sodium sulfate was removed by filtration, and the chloroform concentrated under reduced pressure and dried. After dissolving the product in 20 ml of methanol while heating to 60° C, the solution obtained was ice-cooled and crystallized. The methanol solution was eliminated by decantation, and after dissolving the crystals in 10 ml of ethanol and 20 ml of n-hexane while heating to 60° C, the solution was ice-cooled while stirring and white crystals deposited. These were filtered off, washed with n-hexane and dried. 8.1 g of white crystals were obtained. Melting point = 123° C - 129° C.
Elemental Analysis for $C_{82}H_{83}O_{41}Cl$:
Calcd.(%): C 49.00; H 5.56; Cl 2.23;
Found (%): C 48.70; H 5.40; Cl 2.18;

(C) Method for manufacturing 2-chloro-2′-methane sulfonyl-4′-nitroazobenzene-hexadeca-acetylmaltopentaoside

To 50 ml of dry dimethylformamide were dissolved 2 g of hexadeca-acetylmaltopentaosyl chloride obtained in (B), 0.8 g of 2-chloro-2′-methanesulfonyl-4′-nitroazobenzene and 1.5 ml of triethylamine, and then were refluxed with heat for 5 hours while stirring. After the reaction, 100 ml of dimethylformamide was additionally added and an excess amount of azobenene dye was extracted three times with an aqueous alkaline solution comprising 10 g of sodium carbonate, 150 ml of water and 25 ml of dimethyl sulfoxide and the organic phase was washed with 300 ml of water, the dimethylformamide phase was separated and dried over anhydrous sodium sulfate, and the solvent was removed by evaporation under reduced pressure to obtain 1.9 g of redish brown crude product. The crude product was used to the following step.

(D) Method fo manufacturing 2-chloro-2′-methanesulfonyl-4′-nitroazobenzenemaltopentaoside

To a solution mixture comprising 2 ml of anhydrous methanol and 7 ml of anhydrous dichloromethane was dissovled 1 g of the compound obtained in the step (C) and futher 1.0 ml of 0.5 N sodium methoxide was added. The mixture was reacted with stirring at room temperature for 10 hours. After reaction, the precipitation was filtrated and dried, followed by washing with a mixutre of methanol and dichloromethane (1:1) and then with dichloromethane. The precipitation thus obtained was dried under reduced pressure to obtain 0.43 g of crude crystal.

The crude crystal was purified with a column chromatography of Bio-gel P-2 (Bio Rad Laboratories,

California, U.S.A.) using water to obtain 0.31 g of the desired product (melting point: 178-183 °C).
Elemental Analysis for $C_{43}H_{60}N_3SO_{30}Cl$
Calcd.(%): C 44.3; H 5.1; Cl 3.6;
Found (%): C 43.6; H 5.3; Cl 3.5;

## EXAMPLE 2

On a colorless transparent smooth polyethylene terephthalate film subbed with gelatin (180 µm thickness), an aqueous solution having the following composition (a) was coated and dried to obtain dry thickness of 7 µm.

Composition (a)

Gelatin 300 g
Surface Surfactant (Surfactant 10G made by Oline Corp.) 5 g
15% latex solution of poly-co(styrene-N-methylmorphorium methylstyrene-divinylbenzene, (monomer molar ratio 55:43:2) 280 g
Water 2150 g
(The solution was adjusted to pH 7.0 using dil. NaOH)

On the gelatin layer thus obtained, an aqueous solution having the following composition (b) was coated and dried to obtain dry thickness of 5 µm.

Compositon (b);

Gelatin 200 g
Surface Surfactant (Surfactant 10G made by Oline Corp.) 5 g
α-Glucosidase 3,500,000IU
Water 2600 g
(The solution was adjusted to pH 7.0 using dil. NaOH)

On the gelatin layer thus obtained, an aqueous solution having the following composition (c) was coated and dried to obtain dry thickness of 3 µm.

Composition (c);

Gelatin 30 g
Surface Surfactant (Surfactant 10G made by Oline Corp.) 4 g
Titanium Dioxide (Anatase type) 20 g
Water 950 g
(The solution was adjusted to pH 7.0 using dil. NaOH)

Over the $TiO_2$/gelatin layer thus obtained, water was supplied uniformly in an amount of about 30 g/m$^2$ to wet the layer and thereon a tricot knitted material (made of polyester, 40 gauge) was laminated with a slight pressure and dried.

An aqueous solution having the following composition (d) was coated uniformly at the coverage of 150 cc/m$^2$ and dried to obtain a integral multilayer analytical element for the measurement of the activity of amylase.

Composition (d);

Compound obtained in Example 1 12 g
Water 1600 g
Potassium phosphate 60 g
Polyvinylpyrrolidone (Average molecular weight 700,000) 140 g
(The solution was adjusted to pH 7.0 using dil. NaOH)

## COMPARATIVE EXAMPLE 1

10 µl each of artificial sera I and II having different amylase activity as samples were spotted respectively on the analytical element described above. Change in reflection density per minute between 3 minutes to 8 minutes after spotting of each sample was measured at the wavelength of 520 nm, maintaining the temperature at 37° C. Thus, amylase activity was obtained based on a caliburation curve which was previously prepared using standard sera.

The results are shown in the following Table 1.

Table 1

| Control Serum | Amylase Activity |
|---|---|
| I | 720 U/l |
| II | 238 U/l |

As is apparent from the results, activity of amylase in artificial sera was measured with a high precision by the analytical element using the compound of the present invention.

While the invention has been described in detail and with reference to specific examples thereof, it will be apparent to one skilled in the art that various changes and modifications can be made therein without departing from the spirit and scope thereof.

**Claims**

1. An azo glycoside composition comprising a compound formula (I) represented by

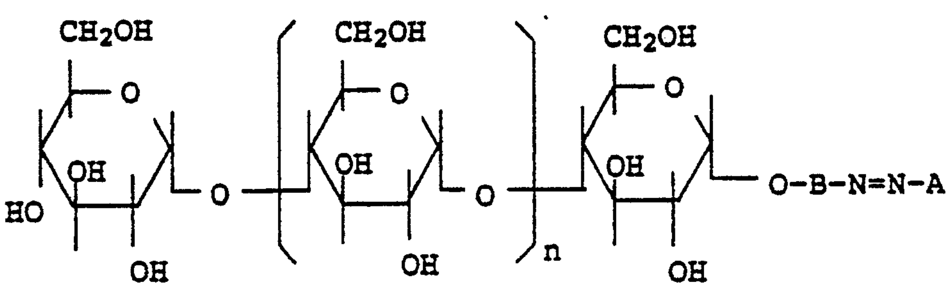

(I)

wherein, A represents a substituted or unsubstituted phenyl group or a substituted or unsubstituted naphthyl group; B represents a substituted or unsubstituted phenylene group; and n represents 0 or an integer of 1 to 8.

2. The composition as claimed in Claim 1, wherein n represents 3, 4 or 5.

3. The composition as claimed in claim 1, wherein said phenylene group is a substituted or unsubstituted 1,4-phenylene group.

4. The composition as claimed in claim 1, wherein said substituted phenyl group and substituted naphthyl group represented by A each is substituted with at least one substituent selected from the group consisting of a halogen atom, a nitro group, an alkane sulfonyl group, a sulfonamide group, a sulfamoyl group, an alkyl group, an alkoxy group, an alkylthio group, a phenoxy group and phenylthio group; and said substituted phenylene group represented by B is substituted with at least one substituent selected from the group consisting of an alkyl group, an alkoxy group, an alkylthio group, an sulfonamido group, a sulfamoyl group, a carbonamido group, a carbamoyl group and a hydroxyl group.

5. The composition as claimed in claim 4, wherein said substituted phenyl group and substituted naphthyl group represented by A each is substituted with at least one substitutent selected from a halogen atom, a nitro group, an alkane sulfonyl group containing at most 4 carbon atoms, a sulfonamido group containing from 1 to 7 carbon atoms, a sulfamoyl group containing from 1 to 7 carbon atoms, an alkyl group containing at most 4 carbon atoms, an alkoxy group containing at most 4 carbon atoms, an alkylthio group containing at most 4 carbon atoms, an alkoxy group containing at most 4 carbon atoms substituted with an alkoxy group containing at most 4 carbon atoms substituted with an alkoxy group, an alkylthio group containing at most 4 carbon atoms subtituted with an alkoxy group, an unsubstituted phenoxy group, an a phenoxy group substituted with at least one halogen atom; and said substituted phenylene group represented by B is substituted with at least one substitutent selected from an alkyl group containing at most 4 carbon atoms, an alkoxy group containing at most 4 carbon atoms, an alkylthio group containing at most 4 carbon atoms, an alkoxy group containing at most 4 carbon atoms substituted with an alkoxy group, an alkylthio group containing at most 4 carbon atoms substituted with an alkoxy group, a sulfonamido group containing at most 7 carbon atoms, a sulfamoyl group containing at most 7 carbon atoms, a carbonamido group containing at most 8 carbon atoms, a carbamoyl group containing at most 8 carbon atoms, and a hydroxyl group.

6. The composition as claimed in calim 1, wherein said -B-N=N-A moiety is represented by formula (II):

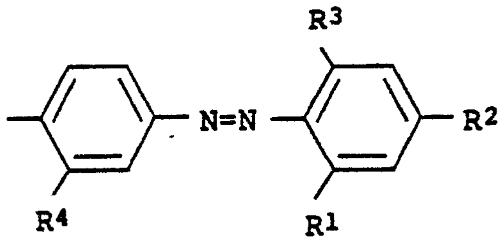

(II)

wherein $R^1$ and $R^2$, which may be the same or different, each represents a hydrogen atom, a chlorine atom, a bromide atom, a nitro group, a methane sulfonyl group, or an ethane sulfonyl group; $R^3$ represents a hydrogen atom, a chlorine atom or a bromine atom; and $R^4$ represents a hydrogen atom, a chlorine atom, a bromine atom, or a lower alkyl group, or formula (III):

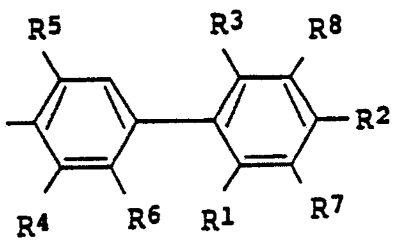

(III)

wherein $R^1$, $R^2$, $R^3$ and $R^4$ each presents the same meaning as defined for that in general formula (I), $R^5$ represents the same meaning as defined for $R^4$. $R^7$ and $R^8$ each represents the same meaning as defined for $R^1$. $R^6$ represents a hydrogen atom, a halogen atom or -NHCOR$^{61}$ (R$^{61}$ represents a lower alkyl group having 1 to 4 carbon atoms or a phenyl group.)

7. A method for producing an azo glycoside composition comprising a compound of formula (I)

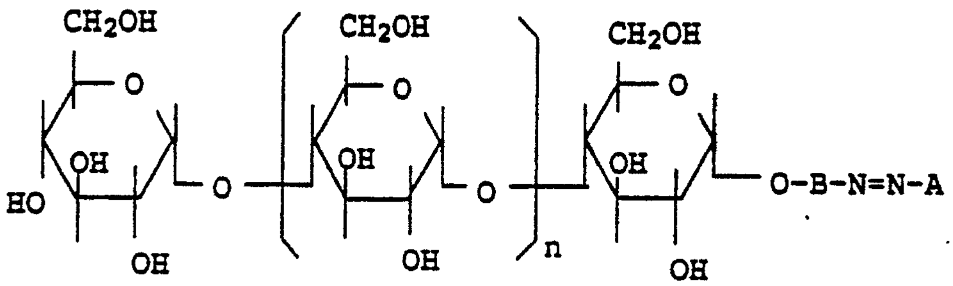

(I)

wherein, A represents a substituted or unsubstituted phenyl group or a substituted or unsubstituted naphthyl group; B represents a substituted or unsubstituted phenylene group; and n represents 0 or an integer of 1 to 8, comprising the steps of

a) acylating an oligosaccharide represented by formula (Ia):

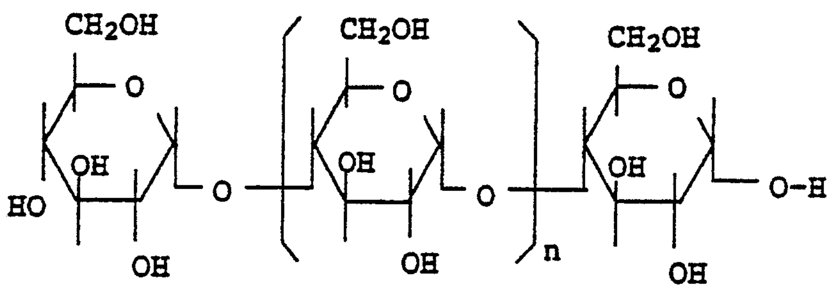

(Ia)

wherein n is 0 or an integer of 1 to 8, with an organic anhydride represented by formula (Ib):

$(RCO)_2O$ (Ib)

wherein R represents a lower alkyl group, to produce a compound represented by formula (Ic):

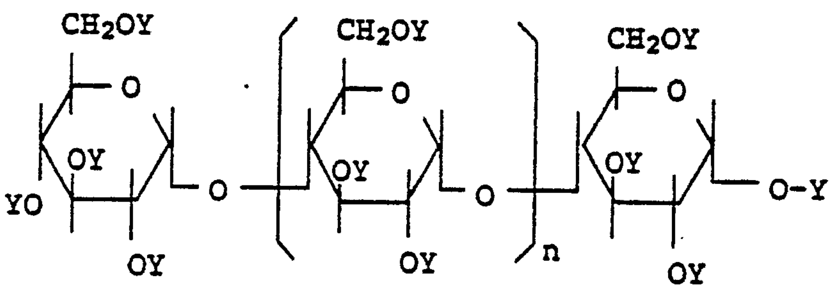

(Ic)

wherein Y represents COR:

(b) halogenating said compound represented by formula (Ic) to provide a compound represented by formula (Id):

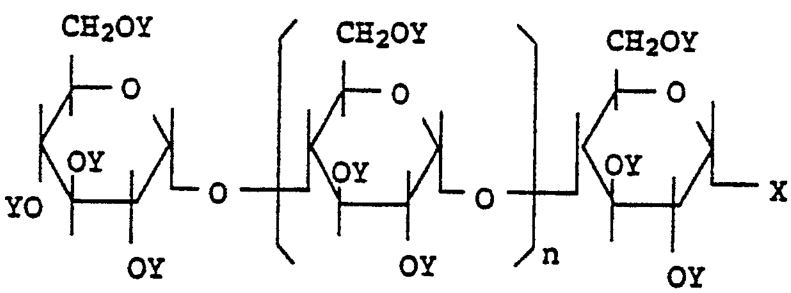

(Id)

wherein X represents a halogen:

(c) reacting said compound represented by formula (Ib) with an azo compound represented by formula (Ie):

HO-B-N = N-A (Ie)

wherein, A represents a substituted or unsubstituted phenyl group or a substituted or unsubstituted naphthyl group and B represents a substituted or unsubstituted phenylene group to produce a compound represented by formula (If):

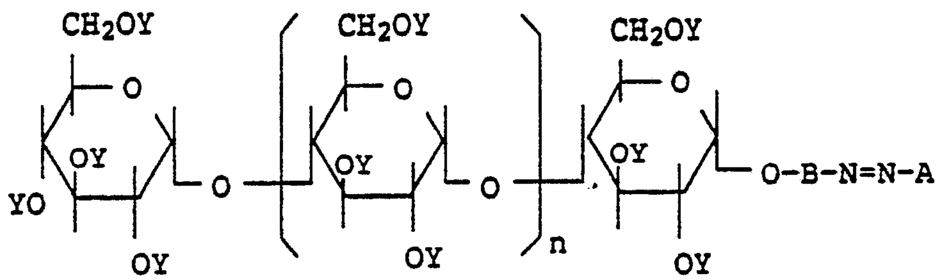

(If)

and

(d) deacylating said compound represented by formula (If) to produce a compound represented by formula (I):

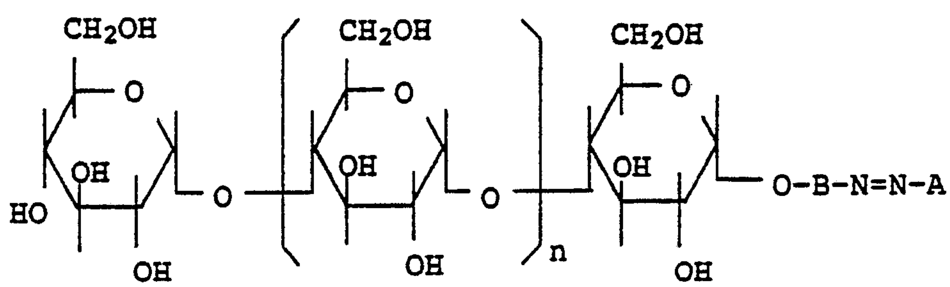

(I)

8. The method as claimed in claim 7, wherein said acylating step (a) is conducted in the presence of an anhydrous alkali metal salt of an organic acid; said organic anhydride represented by $(RCO)_2O$ is selected from acetic anhydride, propionic anhydride and butyric anhydride; said organic anhydride is present in an amount of form about 5 to 50 part by weight per 1 part by weight of said oligosaccharide and siad anhydrous alkali metal salt of an organic acid is present in an amount of from about 0.5 to 3 parts by weight per 1 part by weight of said oligosaccharide, and acylating reaction being conducted at a temperature of from about 90 to 140° C.

9. The method as claimed in claim 8, wherein said organic anhydride is present in an amount of from about 7 to 15 parts by weight per 1 part by weight of said oligosaccharide, said anhydrous alkali metal salt of an organic acid is present in an amount of from about 0.5 to 1.0 parts by weight per 1 part by weight of said oligosaccharide, and said acylating reaction is conducted at a temperature of from about 100 to 110° C.

10. The method as claimed in claim 7, wherein said halogenating step (b) is performed by reacting said compound represented by formula (Ic) with at least one of an anhydrous hydrogen halide, aluminum chloride, phosphorous pentachloride, titanium tetrachloride and stannic chloride.

11. The method as claimed in claim 10, wherein said halogenating is performed by reacting said compound represented by formula (Ic) with an anhydrous titanium tetrahalide in the presence of a non-aqueous solvent of low polarity.

12. The method as claimed in claim 11, wherein said anhydrous titanium tetrahalide is selected from the group consisting of titanium tetrachloride, titanium tetrabromide and titamium tetraiodide, said anhydrous titanium tetrahalide being present in an amount of from about 1 to 20 mol per mol of said compound represented by formula (Ic).

13. The method as claimed in claim 12, wherein said anhydrous titanium halide is present in an amount of from about 3 to 8 mol per 1 mol of said compound represented by formula (Ic).

14. The method as claimed in claim 7, wherein in step (c) said azo compound is present in an amount of from about 1 to 20 mol per mol of said compound represented by formula (Id).

15. The method as claimed in claim 14, wherein said azo compound is present in an amount of from about 1.2 to 3.0 mol per mol of said compound represented by formula (Id).

16. The method as claimed in claim 15, wherein said azo compound is present as an inorganic salt or an organic salt of said azo compound.

17. The method as claimed in claim 7, wherein said deacylating step (d) is performed in the presence of an alkali metal alkoxide and a solvent selected from dry methanol, a methanol solution of anhydrous ammonia, and a mixture of druy methanol and a non-aqueous solvent of low polarity.

18. An analytical element for a measurement of an activity of polysaccharide hydrolases comprising a support, a liquid spreading layer which can receive or absorb a sample and a reagent layer containing an azo glycoside composition comprising a compound formula (I) represented by

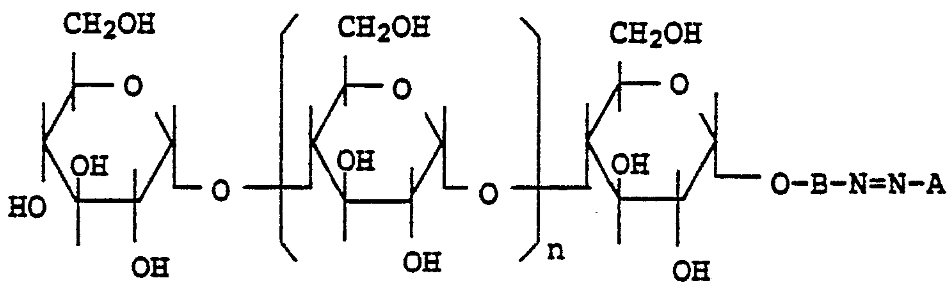

(I)

wherein, A represents a substituted or unsubstituted phenyl group or a substituted or unsubstituted naphthyl group, B represents a substituted or unsubstituted phenylene group; and n represents 0 or an integer of 1 to 8.

19. An analytical element for a measurement of an activity of polysaccharide hydrolases comprising a support, a detecting layer and a liquid spreading layer which can receive or absorb a sample and contains an azo glycoside composition comprising a compound formula (I) represented by

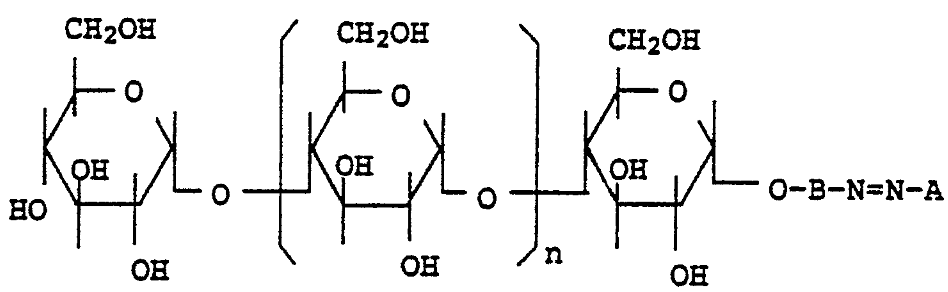

(I)

wherein, A represents a substituted or unsubstituted phenyl group or a substituted or unsubstituted naphthyl group, B represents a substituted or unsubstituted phenylene group: and n represents 0 or an integer of 1 to 8.